(19) Europäisches Patentamt
European Patent Office
Office européen des brevets

(11) Veröffentlichungsnummer: **0 226 984 B1**

(12) **EUROPÄISCHE PATENTSCHRIFT**

(45) Veröffentlichungstag der Patentschrift: **06.06.90**

(51) Int. Cl.[5]: **A61K 7/043**

(21) Anmeldenummer: **86117377.1**

(22) Anmeldetag: **13.12.86**

(54) **Antimykotisch wirksamer Nagellack.**

(30) Priorität: **19.12.85 DE 3544983**

(43) Veröffentlichungstag der Anmeldung: **01.07.87 Patentblatt 87/27**

(45) Bekanntmachung des Hinweises auf die Patenterteilung: **06.06.90 Patentblatt 90/23**

(84) Benannte Vertragsstaaten: **AT BE CH DE ES FR GB GR IT LI LU NL SE**

(56) Entgegenhaltungen:
**EP-A- 0 055 397**
**EP-A- 0 140 325**
**DE-A- 3 140 954**
**US-A- 4 185 106**
**US-A- 4 250 164**

**CHEMICAL ABSTRACTS, Band 92, Nr. 6, Februar 1980, Seite 331, Zusammenfassung Nr. 47183b, Columbus, Ohio, US; W. DITTMAR et al.: "Ciclopirox - substance with aspects for mycology and cosmetics"**

(73) Patentinhaber: **HOECHST AKTIENGESELLSCHAFT, Postfach 80 03 20, D-6230 Frankfurt am Main 80(DE)**

(72) Erfinder: **Bohn, Manfred, Dr., Schweriner Weg 10, D-6238 Hofheim am Taunus(DE)**
Erfinder: **Dittmar, Walter, Dr., Uhlandstrasse 10, D-6238 Hofheim am Taunus(DE)**
Erfinder: **Pell, Heinz Georg, Dr., Homburger Strasse 52, D-6350 Bad Nauheim(DE)**
Erfinder: **Futterer, Eberhard, Dr., Im Stückes 50, D-6233 Kelkheim (Taunus)(DE)**
Erfinder: **Kraemer, Karl, Dr., Im Buchenhain 37, D-6070 Langen(DE)**

**EP 0 226 984 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Pilzerkrankungen der Nägel (Onychomykosen) sind hartnäckige Krankheitsformen, die bisher nicht befriedigend behandelt werden konnten. Unter dem Begriff Onychomykosen werden verschiedene Typen von Nagelmykosen zusammengefaßt, von denen die durch Dermatophyten verursachten am schwierigsten zu behandeln sind, während die durch Hefepilze verursachten Nagelmykosen bisher am ehesten erfolgreich therapiert werden konnten.

Die Tücke der durch Dermatophyten bedingten Onychomykosen besteht auch darin, daß sie erheblich zur Verbreitung infektiöser Pilze beitragen. Zu ihrer Behandlung hat man bisher, allerdings ohne durchschlagenden Erfolg, verschiedene Wege beschritten.

Eine Behandlungsmethode, die systemische, bestand darin, daß man pilzhemmende Mittel oral verabreichte. Dies erforderte eine Langzeitbehandlung, die erfahrungsgemäß zu Intoxikationen führen kann.

Eine andere Methode besteht darin, die Nägel chirurgisch oder durch Einwirkung von Chemikalien zu entfernen und zu hoffen, daß gesunde, nicht befallene Nägel nachwachsen. Diese Methode ist naturgemäß sehr aggressiv und gibt außerdem auch keine Gewähr, daß die Nägel in der natürlichen Form nachwachsen; vielmehr sind die nachwachsenden Nägel häufig verunstaltet.

Eine dritte, aber schonende Methode besteht darin, daß man die Nägel lokal mit spezifischen, antimykotisch wirksamen Stoffen behandelt. Hierbei hat man die unterschiedlichsten Behandlungsmethoden versucht. So hat man in einer kombinierten Behandlung die Nägel zunächst mit Lösungen der antimykotisch wirksamen Stoffe behandelt und nachts jeweils Cremeverbände angelegt. Auch diese Behandlungsmethode ist für den Patienten naturgemäß sehr unerfreulich und psychisch belastend. Einmal ist die Behandlung der Nägel mit Lösungen mehrmals täglich erforderlich. Zum anderen müssen sie vor allem nachts mit Verbänden versehen werden. Ferner ist ein ständiges Abfeilen der erkrankten Nägel notwendig, was sowohl mühsam ist als auch zur Verbreitung der Erreger beiträgt. Dies alles führt dazu, daß die gewöhnlich vielmonatige Behandlung von den Patienten vielfach nicht durchgehalten wird, sie vielmehr entmutigt und nachlässig werden und daß damit der Therapieerfolg ausbleibt. Der Behandlungserfolg wird bei dieser Methode weiterhin dadurch beeinträchtigt, daß die Lösungen und Cremes gewöhnlich mit Wasser mischbar bzw. hydrophil sind und daher beim Waschen, Baden und Duschen von der Nageloberfläche wieder entfernt bzw. aus dem Nagel herausgelöst werden können, mithin also danach wieder neu aufgetragen werden müssen.

Man hat daher große Hoffnungen in eine völlig andere Methode gesetzt, nämlich in die Behandlung mit einem Nagellack, der den antimykotisch wirksamen Stoff Sulbentin, eine Thiadiazin-Verbindung, enthält. Obwohl diese Methode bereits seit etwa 20 Jahren praktiziert wird, hat sie keinen allgemeinen Eingang in die Therapie gefunden, da mit diesen Nagellacken praktisch nur leichtere Nagelmykosen bekämpft werden können. Ein zufriedenstellender Erfolg blieb dieser Zubereitung vermutlich auch mangels ausreichender Bioverfügbarkeit des Wirkstoffs aus dem nach dem Trocknen des Lackes vorliegenden Feststoffsystem versagt.

Daher hat man viele Fälle, insbesondere die schwereren, nach wie vor mit den oben beschriebenen chirurgischen oder chemischen Methoden bzw. mit der kombinierten Lösungs- und Creme-Therapie behandelt.

Es wurde nun gefunden, daß man Nagelmykosen mit durchschlagendem Erfolg behandeln bzw. den Befall verhindern kann, wenn man den erfindungsgemäßen Nagellack auf die Nägel, insbesondere auf die erkrankten Nägel aufträgt.

Gegenstand der Erfindung ist ein antimykotisch wirksamer Nagellack, enthaltend

a) ein 1-Hydroxy-2-pyridon der allgemeinen Formel I

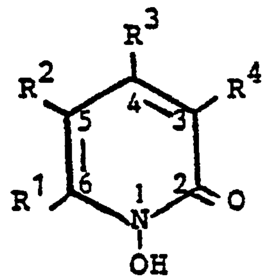

(I)

in der $R^1$ einen gesättigten Kohlenwasserstoffrest mit 6–9 Kohlenstoffatomen,
einer der Reste $R^2$ und $R^4$ ein Wasserstoffatom und der andere Wasserstoff, Methyl oder Ethyl und $R^3$ einen Alkylrest mit 1 oder 2 C-Atomen bedeutet, in freier oder in Salz-Form als antimykotisch wirksamen Stoff,

b) einen wasserunlöslichen Filmbildner,

c) ein physiologisch unbedenkliches Lösemittel, sowie gegebenenfalls

d) in Kosmetika gebräuchliche Zusätze.

Der Begriff "gesättigt" bezeichnet hierbei solche Reste, die keine aliphatischen Mehrfachbindungen, also keine äthylenischen oder acetylenischen Bindungen enthalten.

Mit dem erfindungsgemäßen Nagellack läßt sich bei der Behandlung von Nagelmykosen eine durchgreifende Heilung erzielen, wobei der Nagel gewöhnlich ohne Deformierung nachwächst. Im Hinblick auf die bisherigen schlechten Therapieerfahrungen ist dies ein überaus wichtiger Befund.

Der erfindungsgemäße Nagellack eignet sich auch zur prophylaktischen Anwendung gegen Nagelmykosen, wobei ein ausreichend hohes Wirkstoffdepot im Nagel erreicht wird, so daß es im Falle einer Pilzkontamination nicht zum Ausbruch einer durch Pilze hervorgerufenen Nagelerkrankung kommt.

Der Gehalt an Wirkstoff in dem erfindungsgemäßen Nagellack ist von der Struktur eines jeden Wirkstoffs und damit von dessen Freigabe aus dem Lackfilm, seinem Penetrationsverhalten im Nagel und seinen antimikrobiellen Eigenschaften abhängig.

In dem erfindungsgemäßen Nagellack, also der Lösemittel enthaltenden Anwendungsform, ist der Wirkstoff im allgemeinen in einer Menge von 0,5 bis 20, vorzugsweise 2 bis 15 Gewichtsprozent enthalten. Der Mindestgehalt an Wirkstoff in den medizinischen Nagellacken, also denen zur Behandlung, beträgt meistens 4 Gewichtsprozent; die zur Prophylaxe verwendeten Nagellacke enthalten meistens weniger als 4 und zweckmäßig mindestens 1 Gewichtsprozent Wirkstoff. Jeweils bezogen auf die Menge der nicht flüchtigen Bestandteile, das ist die Summe der Filmbildner, der gegebenenfalls vorhandenen Pigmente, Weichmacher und anderen nicht flüchtigen Zusätze sowie Wirkstoff, ist in den erfindungsgemäßen Nagellacken der Wirkstoff im allgemeinen in einer Menge von 2 bis 80, vorzugsweise von 10 bis 60 und insbesondere von 20 bis 40 Gewichtsprozent enthalten.

In der Formel I ist der Kohlenwasserstoff-Rest $R^1$ ein Alkyl- oder Cyclohexylrest, der auch über eine Methylen- oder Äthylengruppe an den Pyridonring gebunden sein oder eine Endomethylengruppe enthalten kann. $R^1$ kann auch einen aromatischen Rest darstellen oder enthalten, der jedoch vorzugsweise über wenigstens ein aliphatisches C-Atom an den Pyridonrest gebunden ist.

Als geeignete Wirkstoffe seien beispielsweise genannt 1-Hydroxy-4-methyl-6-n-hexyl-, -6-isohexyl-, -6-n-heptyl- oder -6-iso-heptyl-2-pyridon, 1-Hydroxy-4-methyl-6-octyl- oder -6-iso-octyl-2-pyridon, insbesondere als 1-Hydroxy-4-methyl-6-(2,4,4-trimethylpentyl)-2-pyridon, 1-Hydroxy-4-methyl-6-cyclohexyl-2-pyridon, 1-Hydroxy-4-methyl-6-cyclohexylmethyl- oder -6-cyclohexyläthyl-2-pyridon, wobei der Cyclohexylrest jeweils noch einen Methylrest tragen kann, 1-Hydroxy-4-methyl-6-(2-bicyclo [2,2,1] heptyl)-2-pyridon, 1-Hydroxy-3,4-dimethyl-6-benzyl- oder -6-dimethylbenzyl-2-pyridon und 1-Hydroxy-4-methyl-6-(β-phenyl-äthyl)-2-pyridon.

Die erfindungsgemäßen Nagellacke enthalten außer dem in einem Lösemittel oder Lösemittelgemisch gelösten Wirkstoff als notwendige Bestandteile noch einen oder mehrere Filmbildner, die nach dem Trocknen der Zubereitung einen wasserunlöslichen Film auf dem Nagel bilden.

Als Filmbildner eignen sich z.B. Stoffe auf der Basis von Cellulosenitrat oder physiologisch unbedenkliche Polymerisate, wie sie z.B. in Kosmetika üblich sind, vorzugsweise als Gemisch mit Cellulosenitrat. Genannt seien z.B. Polyvinylacetat und partiell verseiftes Polyvinylacetat, Mischpolymerisate aus Vinylacetat einerseits und Acrylsäure oder Crotonsäure oder Maleinsäuremonoalkylester andererseits, ternäre Mischpolymerisate aus Vinylacetat einerseits und Crotonsäure und Vinylneodecanoat, oder Crotonsäure und Vinylpropionat andererseits, Mischpolymerisate aus Methylvinyläther und Maleinsäuremonoalkylester, insbesondere als Maleinsäuremonobutylester, Mischpolymerisate aus Fettsäurevinylester und Acrylsäure oder Methacrylsäure, Mischpolymerisate aus N-Vinylpyrrolidon, Methacrylsäure und Methacrylsäurealkylester, Mischpolymerisate aus Acrylsäure und Methacrylsäure oder Acrylsäurealkylester oder Methacrylsäurealkylester, Polyvinylacetale und Polyvinylbutyrale, alkylsubstituierte Poly-N-vinylpyrrolidone, Alkylester aus Mischpolymerisaten aus Olefinen und Maleinsäureanhydrid und Umsetzungsprodukte von Kolophonium mit Acrylsäure. In den Estern sind die Alkylreste gewöhnlich kurzkettig und haben meistens nicht mehr als vier C-Atome.

Als physiologisch unbedenkliche Lösemittel kommen Stoffe wie in Kosmetika übliche Kohlenwasserstoffe, halogenierte Kohlenwasserstoffe, Alkohole, Äther, Ketone und Ester in Betracht, insbesondere Essigsäureester von einwertigen Alkoholen, wie Äthyl- und Butylacetat, gegebenenfalls im Gemisch mit aromatischen Kohlenwasserstoffen, wie Toluol, und/oder Alkoholen, wie Äthanol oder Isopropanol.

Die Kombination der Lösemittel ist bekanntlich von entscheidender Bedeutung für die Trockenzeit, Verstreichbarkeit und andere wichtige Eigenschaften des Lackes bzw. des Lackfilms. Das Lösemittelsystem besteht vorzugsweise aus einer optimalen Mischung aus Niedrigsiedern (= Lösemittel mit einem Siedepunkt bis 100 °C) und Mittelsiedern (= Lösemittel mit einem Siedepunkt bis 150 °C, gegebenenfalls mit einem kleinen Anteil Hochsiedern (= Lösemittel mit einem Siedepunkt bis 200 °C).

Die erfindungsgemäßen Nagellacke können weiterhin in Kosmetika gebräuchliche Zusätze enthalten, wie Weichmacher auf Phthalat- oder Campherbasis, Farbstoffe bzw. Farbpigmente, Perlglanzmittel, Sedimentationsverzögerer, Sulfonamidharze, Silikate, Riechstoffe, Netzmittel, wie Natriumdioctylsulfosuccinat, Lanolinderivate, Lichtschutzmittel, wie 2-Hydroxy-4-methoxybenzophenon, antibakteriell wirksame Substanzen und Stoffe mit keratolytischer und/oder keratoplastischer Wirkung, wie Ammoniumsulfit, Ester und Salze der Thioglykolsäure, Harnstoff, Allantoin, Enzyme und Salizylsäure.

Gefärbte bzw. pigmentierte Nagellacke haben z.B. den Vorteil, daß die erfindungsgemäße Zubereitung dem Schönheitsempfinden des Patienten angepaßt werden kann.

Die Herstellung des Nagellackes erfolgt in üblicher Weise durch Zusammengeben der einzelnen Komponenten und eine - soweit erforderlich - der jeweiligen Zubereitung angepaßte Weiterverarbeitung.

Die erfindungsgemäßen Nagellacke unterscheiden sich auch grundsätzlich von den aus der europäischen Patentschrift 55 397 bekannten antimykotischen Mitteln, die als Wirkstoffe Azolderivate, insbesondere Imidazol- und Triazol-Derivate, enthalten. Diese antimykotischen Mittel sollen als wasserlöslicher Film aufgetragen werden, eine Depotwirkung aufweisen und eine Kurzzeittherapie ermöglichen. Sie sollen sich auch zur Behandlung von Nagelmykosen eignen und sowohl in Lösungen als auch in Sprays, die nach dem Trocknen einen wasserlöslichen Film bilden, eingesetzt werden. Die Verwendung derartiger wasserlöslicher Bindemittel bewirkt naturgemäß, daß das aufgetragene Mittel bei jedem Waschen mehr oder weniger entfernt wird.

Die Verwendung wasserunlöslicher filmbildender Polymerer gemäß der vorliegenden Erfindung steht in krassem Gegensatz zu der Aussage in der EP-PS 55 397, wonach dann, wenn man anstelle der dort beschriebenen Formulierungen mit wasserlöslichen Polymeren wasserunlösliche Polymere, z.B. "Methacrylate" verwendet, die Mykose verschlimmert wird. Mit Hilfe der erfindungsgemäßen Nagellacke, die nach dem Trocknen wasserunlöslich werdende filmbildende Polymere enthalten, läßt sich die Nagelmykose dagegen erfolgreich behandeln.

Die oberen Hornschichten haben bekanntlich u.a. die biologische Aufgabe, eindringende Fremdstoffe abzuwehren. Die erfindungsgemäßen Zubereitungen unterscheiden sich auch dadurch, und zwar grundsätzlich, von den bisher zur Nagelbehandlung empfohlenen Zubereitungen, daß sie solche Wirkstoffe enthalten, die von den oberen Hornschichten in einem beachtlichen Maße durchgelassen werden und so in der Tiefe eine lang anhaltende Wirkung ausüben. Die Hornpenetration in wirksamer Konzentration ist danach eine von der antimykotischen Eigenschaft abzutrennende Besonderheit der erfindungsgemäß verwendeten Pyridonverbindungen, die es erstmals erlaubt, Nagelmykosen auf einfache und effektive Weise zu behandeln.

Die Wirkung der erfindungsgemäß verwendeten Verbindungen wurde in Penetrationstests an exzidierter verhornter Haut und in klinischen Behandlungsversuchen an Patienten mit Onychomykosen nachgewiesen. Das Prüfverfahren auf Penetrationsvermögen an exzidierter Haut von Schweinen erlaubt es, Verbindungen auf ihr Penetrationsvermögen in wirksamer Konzentration gegenüber verhorntem Gewebe zu prüfen.

Die vorliegende Erfindung wird durch die folgenden Beispiele näher erläutert. Die prozentualen Mengenangaben sind auf das Gewicht bezogen. T bedeutet Gewichtsteile.

Beispiele 1 - 8 - Prüfung der Wirksamkeit

In den Tests auf das Penetrationsvermögen wurde zunächst die Oberfläche von geschorenen Hautstücken mit O,3%igen Lösungen der Verbindungen 1 - 8 und ebenso mit 3 ähnlichen nicht beanspruchten Verbindungen, jeweils gelöst in einem Gemisch aus 1 ml Dimethylsulfoxyd und 9 ml Isopropanol, bei Raumtemperatur behandelt und nach 2 Stunden wieder abgewaschen. Sodann wurde der tiefste Bereich der Hornschicht freigelegt, indem die oberflächlichen Schichten durch 1O aufeinanderfolgende Klebstreifenabrisse entfernt wurden. Die nun freiliegende tiefe Hornschicht wurde mit virulenten Erregern von Nagelmykosen (Dermatophyten) inokuliert. Anhand des Grades der gefundenen Wachstumshemmung wurde auf die Höhe des Penetrationsvermögens geschlossen.

In der Tabelle 1 sind die Ergebnisse dieser Versuche vergleichend zu der Wirksamkeit der Pyridon-Verbindungen gegen den hochvirulenten Hautpilz Trichophyton mentagrophytes 1O9(1OO/25) im In-vitro-Reihenverdünnungstest und im Meerschweinchen-Trichophytie-Modell aufgeführt.

Obwohl bei der Prüfung auf antimycetische Wirksamkeit im Reihenverdünnungstest und auf antimykotische Wirkung im Meerschweinchen-Trichophytie-Modell an oberflächlichen Hornschichten die Verbindungen 1 - 8 ähnliche Ergebnisse wie die Vergleichsverbindungen 1 - 3 zeigen, ergibt sich aus der Wachstumshemmung in dem tiefsten Bereich der Hornschicht von Schweinehaut, daß die Verbindungen 1 - 8 ein wesentlich besseres Penetrationsvermögen aufweisen als die Vergleichsverbindungen 1 - 3.

Beispiele 9 und 1O - Wirksamkeitsprüfung mit Nagellacken

In weiteren Versuchen wurde die wesentlich stärker verhornte menschliche Haut nach dem oben beschriebenen Prüfverfahren mit 3%igen Nagellacken der Verbindungen 9 und 1O und ebenso mit einer ähnlichen nicht beanspruchten Verbindung 4 im Vergleich zu dem bereits früher erwähnten Sulbentin enthaltenden Nagellack 1 bzw. 2 Stunden bei Raumtemperatur behandelt.

Die Lackgrundlage der Beispiele 9 und 1O bzw. des Vergleichs 4 und 5 wurde durch Vermischen folgender Bestandteile hergestellt:

| | |
|---|---|
| Isopropylalkohol | 34,4% |
| Äthylacetat | 34,5% |
| 2-Hydroxy-4-methoxybenzophenon | 0,1% |
| Natriumdioctylsulfosuccinat | 1,0% |
| 50%ige Lösung eines Mischpolymerisates aus Methylvinyläther und Maleinsäuremonobutylester in Isopropylalkohol | 30,0% |

Die in der Tabelle 2 aufgeführten Ergebnisse der Versuche mit Lackzubereitungen an der menschlichen Haut stehen in guter Übereinstimmung mit den Ergebnissen von den mit Wirkstofflösungen an Schweinehaut durchgeführten Untersuchungen. Während die Lacke, die die Verbindungen 9 und 10 enthalten, das Wachstum von Trichophyton mentagrophytes in der tiefen Hornschicht der menschlichen Haut nahezu vollständig bzw. vollständig hemmen, zeigen die die Vergleichsverbindung 4 enthaltenden Lacke - ebenso wie der Sulbentin enthaltende Lack - nur ungenügende Hemmwirkung.

Beispiele 11 - 14

Einige erfindungsgemäße Verbindungen werden wie folgt zu Nagellacken (Beispiele 11 - 13 farblos, Beispiel 14 pigmentiert) verarbeitet. Die farblosen Nagellacke werden durch Lösen der verschiedenen Komponenten in den Lösemitteln hergestellt.

| | |
|---|---|
| 11. Isopropylalkohol | 57,5% |
| Äthylacetat | 33,0% |
| Polyvinylbutyral | 3,8% |
| Cellulosenitrat | 3,1% |
| Dibutylphthalat | 0,6% |
| 1-Hydroxy-4-methyl-6-(2,4,4-trimethylpentyl)-2-pyridon | 2,0% |

| | |
|---|---|
| 12. Isopropylalkohol | 27,0% |
| Äthylacetat | 27,0% |
| 50%ige Lösung eines Mischpolymerisates aus Methylvinyläther und Maleinsäuremonobutylester in Isopropylalkohol | 34,0% |
| 1-Hydroxy-4-methyl-6-cyclohexyl-2-pyridon | 12,0% |

| | |
|---|---|
| 13. Äthanol | 5,0% |
| Äthylacetat | 68,5% |
| Methylacetat | 10,0% |
| Polyvinylacetat (z.B. Mowilith® 30) | 12,5% |
| 1-Hydroxy-4-methyl-6-cyclohexyl-2-pyridon | 4,0% |

14. Durch langsames Einrühren von 10 t eines organisch modifizierten Montmorillonits (z.B. Bentone 27 ®, 27 Kronos Titan GmbH, Leverkusen, Deutschland) in 80 t Toluol und anschließende Zugabe von 8 t Netzmittel (z.B. Anti-Terra-U ®, Byk-Mallinckrodt, Wesel, Deutschland) und 2 t Methanol wurde eine Thixotropierungspaste hergestellt. Weiter stellte man durch Lösen von 22 t Collodiumwolle, butanolfeucht (z.B. Typ E 510, Wolff Walsrode AG, Deutschland), und 8 t Toluolsulfonamidharz (z.B. Santolite MS 80 ®, Monsanto, Mülheim-Ruhr, Deutschland) in einer Mischung aus 3 t Dibutylphthalat, 20 t Äthylacetat, 10 t Butylacetat, 7 t Äthylalkohol und 30 t Toluol einen Klarlack her. Außerdem wurden in einer geeigneten Kugelmühle 40 t DC ROT No. 7 Calciumlack (z.B. Farbpigment C 19021, Sun Chemical Corporation, Pigments Division, Fort Lee, USA) und 60 t Dibutylphthalat zu einer Farbpaste mit einer Teilchengröße unter 1 µm verarbeitet.

Zur Herstellung des pigmentierten Nagellackes wurden 12 t Thixotropierungspaste und 0,8 t Antiabsetzmittel (z.B. MPA 2000 X ®, Kronos Titan GmbH) in 83,7 t Klarlack dispergiert, wobei eine Temperatur von mindestens 38 °C erreicht werden soll. Anschließend wurde 1 t 1-Hydroxy-4-methyl-6-(2,4,4-trimethylpentyl)-2-pyridon in dem thixotropierten Klarlack gelöst und 2,5 t Farbpaste eingerührt. Der fertige Nagellack wurde durch ein 70-µm-Sieb filtriert.

Tabelle I

| Beispiel | Formel I gemäß Patentanspruch I: $R^1$ | $R^2$ | $R^3$ | $R^4$ | Wirkung gegen T. mentagrophytes: in vitro * MHK (µg/ml) | in vivo ** Hemmung (%) auf der Haut | in vitro *** Hemmung (%) in der Hornschicht |
|---|---|---|---|---|---|---|---|
| 1 | $n-C_7H_{15}$ | H | $CH_3$ | H | 1,95 | 89,4 | 97,0 |
| 2 | $\genfrac{}{}{0pt}{}{C_4H_9}{C_2H_5} > CH-$ | H | $CH_3$ | H | 1,95 | 85,6 | 85,8 |
| 3 | $CH_3-C(CH_3)_2-CH_2-CH(CH_3)-CH_2-$ | H | $CH_3$ | H | 1,95 | 91,9 | 99,6 |
| 4 | - 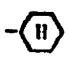 | H | $CH_3$ | H | 1,95 | 88,0 | 98,0 |
| 5 | $-CH_2-CH_2-$ 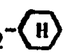 | H | $CH_3$ | H | 1,95 | 88,0 | 81,2 |
| 6 | - 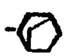 | H | $CH_3$ | H | 1,95 | 88,0 | 79,9 |
| 7 | $-CH_2-$ 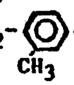 $-CH_3$ | H | $CH_3$ | $CH_3$ | 1,95 | 88,8 | 80,0 |
| 8 | $-CH_2-CH_2-$ 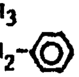 | H | $CH_3$ | H | 1,95 | 88,0 | 81,9 |
| Vergleich | | | | | | | |
| 1 | $-CH(CH_3)_2$ | H | $CH_3$ | H | 1,95 | 75,0 | 35,7 |
| 2 | $-C_{11}H_{23}$ | H | $CH_3$ | H | 3,90 | 100,0 | 9,8 |
| 3 | $-(CH_2)_4-$ 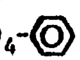 | H | $CH_3$ | H | 3,90 | 88,0 | 24,2 |

* Minimale Hemmkonzentration (MHK) im Reihenverdünnungstest

** Prozentuale Hemmung an der Hautoberfläche im Meerschweinchen-Trichophytie-Modell (W. Dittmar, Mykosen 18 (8), 351 - 361 (1975))

*** Hornpenetration: Prozentuale Wachstumshemmung der in den tiefsten Teil der Hornschicht penetrierten Substanz

Tabelle 2

| Beispiel | Formel I gemäß Patentanspruch I: $R^1$ | $R^2$ | $R^3$ | $R^4$ | Wirkung gegen T. mentagrophytes Hemmung (%) in der tiefen Hornschicht: Schwein (0,3%ige Lösungen), Einwirkungszeit* (min) 120 | Mensch (3%ige Lacke) 60 | 120 |
|---|---|---|---|---|---|---|---|
| 9 | 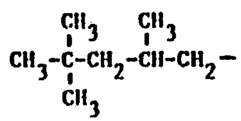 | H | $CH_3$ | H | 99,6 | 99,0 | 99,9 |
| 10 | 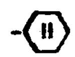 | H | $CH_3$ | H | 98,0 | 100,0 | 100,0 |
| Vergleich | | | | | | | |
| 4 | $-C_{11}H_{23}$ | H | $CH_3$ | H | 9,8 | 51,4 | 49,4 |
| 5 | Leerlack | | | | - | 1,0 | 0,0 |
| 6 | unbehandelt | | | | 0,0 | 0,0 | 0,0 |
| 7 | Sulbentin enthaltender Nagellack | | | | - | 30,0 | 22,4 |

* Zeit zwischen Produktanwendung und Hautabstrippung

## Patentansprüche

1. Antimykotisch wirksamer Nagellack, enthaltend
a) ein 1-Hydroxy-2-pyridon der allgemeinen Formel I

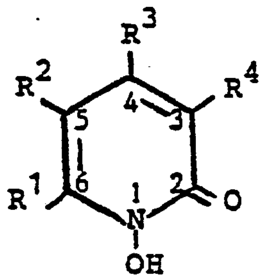

(I)

in der $R^1$ einen gesättigten Kohlenwasserstoffrest mit 6–9 Kohlenstoffatomen,
einer der Reste $R^2$ und $R^4$ ein Wasserstoffatom und der andere Wasserstoff, Methyl oder Ethyl und
$R^3$ einen Alkylrest mit 1 oder 2 C-Atomen bedeutet, in freier oder in Salz-Form als antimykotisch wirksamen Stoff,
b) einen wasserunlöslichen Filmbildner,
c) ein physiologisch unbedenkliches Lösemittel, sowie gegebenenfalls
d) in Kosmetika gebräuchliche Zusätze.

2. Nagellack gemäß Anspruch 1, dadurch gekennzeichnet, daß der Wirkstoff in den Positionen $R^2$ und $R^4$ Wasserstoff enthält.

3. Nagellack gemäß Anspruch 1 oder 2, dadurch gekennzeichnet, daß der Wirkstoff in der Position $R^1$ einen cyclischen Rest enthält.

4. Nagellack gemäß Anspruch 3, dadurch gekennzeichnet, daß $R^1$ vorzugsweise ein Cyclohexylrest ist.

5. Nagellack gemäß Anspruch 1 oder 2, dadurch gekennzeichnet, daß der Wirkstoff in der Position $R^1$ einen Octylrest, insbesondere der Formel $-CH_2-CH(CH_3)-CH_2-C(CH_3)_3$ enthält.

6. Nagellack gemäß einem oder mehreren der Ansprüche 1 bis 5, dadurch gekennzeichnet, daß der Wirkstoff in einer Menge von 2 bis 80, vorzugsweise von 10 bis 60 und insbesondere von 20 bis 40 Gew.-%, bezogen auf die Menge der nicht-flüchtigen Bestandteile enthalten ist.

7. Nagellack gemäß einem oder mehreren der Ansprüche 1 bis 6, dadurch gekennzeichnet, daß er den Wirkstoff in einer Menge von 0,5 bis 20, vorzugsweise von 2 bis 15 Gew.-%, bezogen auf die Menge der flüchtigen und der nicht-flüchtigen Bestandteile, enthält.

8. Verfahren zur Herstellung eines Nagellacks mit einem Gehalt an einem wasserunlöslichen Filmbildner und einem antimykotisch wirksamen Stoff gemäß den Ansprüchen 1 bis 7, dadurch gekennzeichnet, daß man einen wasserunlöslichen Filmbildner mit einem antimykotisch wirksamen Stoff der Formel I (wie in Anspruch 1 definiert) sowie ggf. mit anderen für die Herstellung von Nagellack üblichen Komponenten vermischt.

9. Verwendung einer oder mehrerer Verbindungen der Formel I, wie in Anspruch 1 definiert, als Zusatzmittel in Nagellack(en).

## Claims

1. An antimycotic nail varnish containing
a) a 1-hydroxy-2-pyridone of the formula I

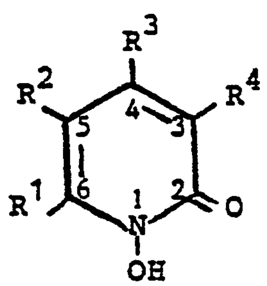

(I)

in which $R^1$ is a saturated hydrocarbon radical having 6–9 carbon atoms, one of the radicals $R^2$ and $R^4$ is a hydrogen atom and the other is hydrogen, methyl or ethyl and

$R^3$ is an alkyl radical having one or two carbon atoms, in the free form or in salt form, as the antimycotic substance,
b) a water-insoluble film-forming agent,
c) a physiologically acceptable solvent, and if appropriate
d) additives customary in cosmetics.

2. A nail varnish as claimed in claim 1, wherein the active compound contains hydrogen in the positions $R^2$ and $R^4$.

3. A nail varnish as claimed in claim 1 or 2, wherein the active compound contains a cyclic radical in the position $R^1$.

4. A nail varnish as claimed in claim 3, wherein $R^1$ is preferably a cyclohexyl radical.

5. A nail varnish as claimed in claim 1 or 2, wherein the active compound contains an octyl radical, in particular of the formula $-CH_2-CH(CH_3)-CH_2-C(CH_3)_3$, in the position $R^1$.

6. A nail varnish as claimed in one or more of claims 1 to 5, which contains the active compound in an amount of 2 to 80, preferably 10 to 60 and in particular 20 to 40% by weight, based on the amount of non-volatile constituents.

7. A nail varnish as claimed in one or more of claims 1 to 6, which contains the active compound in an amount of 0.5 to 20, preferably 2 to 15% by weight, based on the amount of volatile and non-volatile constituents.

8. A process for the preparation of a nail varnish containing a water-insoluble film-forming agent and an antimycotic substance, as claimed in claims 1 to 7, which comprises mixing a water-insoluble film-forming agents with an antimycotic substance of the formula I (as defined in claim 1) and if appropriate with other components customary for the preparation of nail varnish.

9. The use of one or more compounds of the formula I as defined in claim 1 as an additive in a nail varnish.

**Revendications**

1. L'objet de l'invention est un vernis à ongles à action antimycosique, contenant
a) une 1-hydroxy-2-pyridone de formule générale I

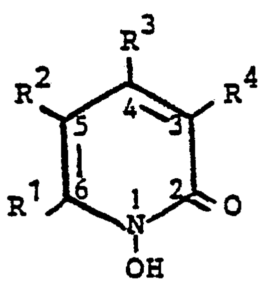

(I)

dans laquelle $R^1$ représente un reste hydrocarboné saturé ayant de 6 à 9 atomes de carbone,
l'un des restes $R^2$ et $R^4$ représente un atome d'hydrogène et l'autre représente un atome d'hydrogène ou le groupe méthyle ou éthyle, et
$R^3$ représente un reste alkyle ayant 1 ou 2 atomes de carbone,
sous forme libre ou sous forme de sel, en tant que substance à action antimycosique,
b) un agent filmogène insoluble dans l'eau,
c) un solvant physiologiquement acceptable, ainsi qu'éventuellement
d) des additifs utilisés habituellement dans des cosmétiques.

2. Vernis à ongles selon la revendication 1, caractérisé en ce que la substance active contient un atome d'hydrogène en positions $R^2$ et $R^4$.

3. Vernis à ongles selon la revendication 1 ou 2, caractérisé en ce que la substance active contient un reste cyclique en position $R^1$.

4. Vernis à ongles selon la revendication 3, caractérisé en ce que $R^1$ est de préférence un reste cyclohexyle.

5. Vernis à ongles selon la revendication 1 ou 2, caractérisé en ce que la substance active contient en position $R^1$ un reste octyle, en particulier de formule $-CH_2-CH(CH_3)-CH_2-C(CH_3)_3$.

6. Vernis à ongles selon l'une quelconque des revendications 1 à 5, caractérisé en ce que la substance active est contenue en une quantité allant de 2 à 80, de préférence de 10 à 60 et en particulier de 20 à 40% en poids, par rapport à la quantité des composants non volatils.

7. Vernis à ongles selon l'une quelconque des revendications 1 à 6, caractérisé en ce qu'il contient la substance active en une quantité allant de 0,5 à 20, de préférence de 2 à 15% en poids, par rapport à la quantité des composants volatils et des composants non volatils.

8. Procédé pour la préparation d'un vernis à ongles ayant une teneur en un agent filmogène insoluble dans l'eau et en une substance à action antimycosique selon les revendications 1 à 7, caractérisé en ce que l'on mélange un agent filmogène insoluble dans l'eau avec une substance active antimycosique de formule I (telle que définie dans la revendication 1) ainsi qu'éventuellement avec d'autres composants usuels pour la préparation de vernis à ongles.

9. Utilisation d'un ou de plusieurs composés de formule I, telle que définie dans la revendication 1, en tant qu'additifs dans un ou des vernis à ongles.